# FASCICULE DE BREVET EUROPEEN

(11) **EP 0 790 243 B1**
(45) Date de publication et mention de la délivrance du brevet: **17.03.1999**
(21) Numéro de dépôt: 97400070.5
(22) Date de dépôt: 15.01.1997
(51) Int. Cl.: C07D 251/70, A61K 7/42

(54) **Dérivés de s-triazine en tant que filtres UV**
S-Triazinederivate als UV-Filter
Derivatives of s-triazine as UV filters

(30) Priorité: 12.02.1996 FR 9601692
(43) Date de publication de la demande: 20.08.1997
(73) Titulaire: L'OREAL, 75008 Paris (FR)
(72) Inventeur: Richard, Hervé, 93420 Villepinte (FR); Leduc, Madeleine, 75011 Paris (FR); Lagrange, Alain, 77700 Coupvray (FR); Plessix, Hervé, 92340 Bourg La Reine (FR)
(74) Mandataire: Miszputen, Laurent

(56) Documents cités:
- EP-A- 0 507 691
- EP-A- 0 507 692
- DE-A- 4 105 923
- CHEMICAL ABSTRACTS, vol. 125, no. 8, 19 Août 1996 Columbus, Ohio, US; abstract no. 99778u, DOUARRE,L. ET AL: "Photochemical study of substituted s-triazines" XP002032513 & J. PHOTOCHEM.PHOTOBIOL. A, vol. 96, no. 1-3, - 1996 pages 71-78,

## Description

La présente invention concerne de nouveaux dérivés insolubles de s-triazine portant des groupements benzalmalonates et/ou phénylcyanoacrylates, leur procédé de préparation et leurs utilisations sous forme particulaire en tant que filtres UV, notamment dans le domaine cosmétique.

La présente invention concerne également l'utilisation de ces nouveaux composés pour la protection de la peau et/ou des cheveux contre le rayonnement ultraviolet, ou pour la protection de toute autre matière sensible aux UV (verres minéraux ou organiques, plastiques ou autre).

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

Un grand nombre de composés ont déjà été proposés comme filtres solaires, sous la forme essentiellement de filtres organiques solubles ou de composés inorganiques insolubles. Ces filtres doivent pouvoir absorber ou bloquer les rayons nocifs du soleil tout en restant inoffensifs pour l'utilisateur.

A cet égard, et afin de limiter les éventuels risques d'allergie sur la peau engendrés par les filtres organiques du fait de leur solubilité, on utilise de plus en plus, pour filtrer les rayons UV, des pigments minéraux tels que l'oxyde de zinc ou encore l'oxyde de titane. Cependant, ces pigments minéraux présentent l'inconvénient d'être sensibles au rayonnement solaire (phénomène connu sous le nom de photobleuissement). Par ailleurs, à quantités équivalentes, ces pigments minéraux sont moins efficaces dans la protection UV que les filtres organiques susmentionnés.

Or, à la suite d'importantes recherches menées dans le domaine de la photoprotection évoqué ci-dessus, la Demanderesse a maintenant découvert de nouveaux filtres UV, insolubles, non minéraux, capables d'absorber à la fois dans l'UV-A et l'UV-B, et qui présentent l'avantage de cumuler à la fois des propriétés de diffusion, puisque ce sont des pigments organiques solides, et d'absorption.

Cette découverte est à la base de l'invention.

Ainsi, selon la présente invention, il est maintenant proposé de nouveaux composés répondant à la formule suivante (I) : 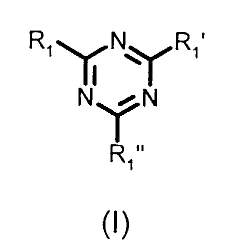 dans laquelle :
- R₁, R₁' et R₁", qui peuvent être identiques ou différents, sont choisis parmi les radicaux monovalents de formules A ou B suivantes : 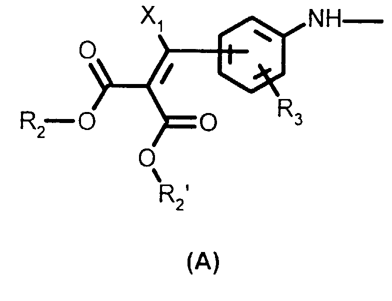 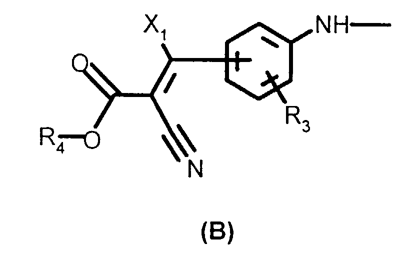 dans lesquelles :
- R₂ et R₂', qui peuvent être identiques ou différents, désignent un radical alkyle en C₁-C₃ linéaire ou ramifié,
- R₃ désigne un atome d'hydrogène, un radical alkyle en C₁-C₄ linéaire ou rami fié ou un radical alkoxy en C₁-C₄,
- R₄ désigne un radical alkyle en C₁-C₁₂ linéaire ou ramifié,
- X₁ représente un atome d'hydrogène ou un radical phényle éventuellement substitué par un halogène ou par un radical alkyle en C₁-C₄ ou par un radical alcoxy en C₁-C₄.

Des composés préférés de la présente invention sont ceux répondant à la formule (I) ci-dessus, les radicaux R₁, R₁" et R₁" étant identiques.

Préférentiellement encore, les composés de la présente invention sont ceux répondant à la formule (I) ci-dessus, les radicaux R₁, R₁" et R₁" étant identiques et désignant chacun un radical de formule A.

Dans une autre forme préférée de réalisation de l'invention, les composés de la présente invention sont ceux répondant à la formule (I) ci-dessus, les radicaux R₁, R₁'et R₁" étant identiques et désignant chacun un radical de formule B.

On notera ici que des dérivés de triazine à fonction(s) benzalmalonate(s) et/ou benzylidène camphre(s) et/ou cinnamate(s) ont déjà été décrits dans les demandes de brevet EP-A-0 507 691, EP-A-0 507 692 et DE-A-41 05 923 et l'article CA : 99778u ; vol 125; N°8 ; 19/08/1996. Toutefois, ces composés de l'art antérieur sont solubles et, par ailleurs, n'absorbent que dans l'UV-A.

Au contraire, et comme indiqué précédemment, les dérivés de l'invention sont des composés insolubles et capables d'absorber simultanément dans l'UV-A et dans l'UV-B. Les groupements benzalmalonates et phénylcyanoacrylates sont des motifs filtrants qui absorbent généralement le rayonnement UV-A les nouveaux composés insolubles conformes à la présente invention, substitués par des groupements benzalmalonates et/ou phénylcyanoacrylates, présentent l'avantage inattendu et surprenant d'absorber à la fois dans l'UV-A et dans l'UV-B.

Par ailleurs, outre leurs propriétés filtrantes et dispersantes, ces nouveaux dérivés s-triaziniques présentent une bonne stabilité chimique et photochimique. Du fait de leur insolublité, ils présentent peu de risques de pénétration dans l'épiderme. Ces composés sont donc tout indiqués pour la préparation de compositions destinées à la protection solaire de la peau et des cheveux.

Par composés insolubles ou substantiellement insolubles, on entend, au sens de la présente invention, des composés dont la solubilité dans l'eau est inférieure à 0,1 % en poids, dont la solubilité dans l'huile de vaseline est inférieure à 1% en poids, et enfin, dont la solubilité dans un mélange d'esters de triglycérides tel que le « Miglyol 812 » commercialisé par la société Dynamit Nobel est inférieure à 2%, également en poids.

La présente invention a également pour objet un procédé de préparation des composés de formule (I) définis ci-avant.

Ainsi, les composés de formule (I) peuvent être obtenus selon le schéma réactionnel ci-dessous : 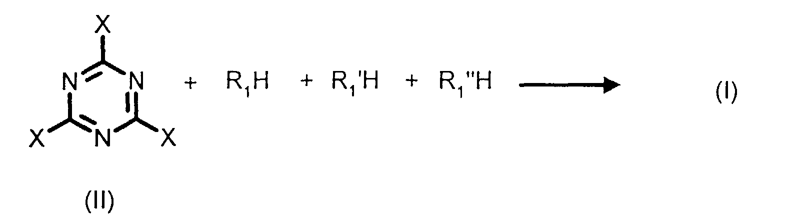 où R₁, R₁" et R₁" répondent aux définitions ci-dessus et X représente un halogène, en particulier le chlore ou le brome.

Dans le cas où R₁, R₁'et R₁" sont différents, les composés R₁H, R₁'H et R₁"H peuvent être introduits dans le mélange réactionnel séparément et l'un après l'autre. Ainsi, dans un premier temps, R₁'H est introduit lorsque R₁H a complètement réagi avec le composé de formule (II). Dans un deuxième temps, R₁"H est introduit lorsque R₁'H a complètement réagi avec le composé de formule (II) monosubstitué par R1.

Les composés R₁H (respectivement R₁'H et R₁"H), où R₁ (respectivement R₁' et R₁") désigne un radical de formule A, peuvent être préparés selon des méthodes connues décrites notamment dans le brevet GB-1 064 116.

Les composés R₁H (respectivement R₁'H et R₁"H), où R₁ (respectivement R₁" et R₁") désigne un radical de formule B, peuvent être préparés selon des méthodes connues décrites notamment dans J. Soc. Dyers Colour. (1977), 93, p126-133.

Les réactions ci-dessus peuvent être effectuées éventuellement en présence d'un solvant (toluène, xylène ou acétone/eau).

Parmi les composés R₁H ci-dessus, on peut citer plus particulièrement les composés suivants :
- 4-amino benzalmalonate de diméthyle,
- 4-amino benzalmalonate de diéthyle,
- 4-amino benzalmalonate de di-n-propyle,
- 4-amino benzalmalonate de diisopropyle,
- α-cyano-4-aminocinnamate d'éthyle,
- α-cyano-4-aminocinnamate d'isopropyle,
- α-cyano-4-aminocinnamate d'éthyl-2-hexyle.
et parmi les composés de formule (I), on peut citer plus particulièrement les composés suivants :
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(α-cyano-4-aminocinnamate d'éthyle)-s-triazine.

Ces nouveaux dérivés de s-triazine insolubles ou substantiellement insolubles peuvent être amenés sous une forme particulaire convenable par tout moyen ad-hoc tel que notamment broyage à sec ou en milieu solvant, tamisage, atomisation, micronisation, pulvérisation. Ils peuvent ensuite être utilisés comme pigments pour la protection solaire de la peau humaine et des cheveux. Ils peuvent également être utilisés comme agents protecteurs de la lumière dans l'industrie des plastiques, du verre (emballage, verres optiques, notamment pour lunetterie) et autres.

La présente invention a également pour objet une composition destinée à protéger une matière sensible au rayonnement ultraviolet, en particulier au rayonnement solaire, comprenant une quantité efficace d'au moins un composé de formule (I).

Plus particulièrement, lorsque la matière sensible à protéger est la peau et/ou les cheveux, cette composition se présente sous la forme d'une composition cosmétique comprenant, dans un support cosmétiquement acceptable, une quantité efficace d'au moins un composé de formule (I).

De préférence, les composés selon l'invention sont utilisés, dans les compositions cosmétiques conformes à l'invention, sous forme particulaire, la taille moyenne des particules étant inférieure à 20 µm.

Le ou les composés de formule (I) peuvent être présents dans la composition cosmétique selon l'invention dans des proportions comprises entre 0,1 et 20% en poids, par rapport au poids total de la composition, de préférence entre 0,1 et 15%.

La composition cosmétique de l'invention peut être utilisée comme composition protectrice de l'épiderme humain ou des cheveux ou comme composition antisolaire.

Les compositions selon l'invention peuvent bien entendu contenir un ou plusieurs filtres solaires complémentaires actifs dans l'UVA et/ou l'UVB (absorbeurs), hydrophiles ou lipophiles. Ces filtres complémentaires peuvent être notamment choisis parmi les dérivés cinnamiques, les dérivés salicyliques, les dérivés du camphre, les dérivés de triazine autres que les composés conformes à la présente invention, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions selon l'invention peuvent encore contenir des pigments ou bien encore des nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir le ou les éventuels composés complémentaires cités ci-dessus et/ou leurs quantités de manière telle que les propriétés avantageuses attachées intrinsèquement au composé conforme à l'invention ne soient pas, ou substantiellement pas, altérées par la ou les adjonctions envisagées.

Lorsque la composition selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Les compositions de l'invention peuvent être préparées selon les techniques bien connues de l'homme de l'art, en particulier celles destinées à la préparation d'émulsions de type huile-dans-eau ou eau-dans-huile.

Lorsque la matière sensible à protéger est un verre organique et/ou minéral ou une matière plastique, les compositions selon l'invention peuvent se présenter sous la forme d'un vernis que l'on applique sur ladite matière sensible afin de la protéger du rayonnement ultraviolet.

La présente invention a encore pour objet l'utilisation d'au moins un composé de formule (I) dans des, ou pour la fabrication de, compositions destinées à protéger des matières sensibles au rayonnement ultraviolet, en particulier au rayonnement solaire.

En particulier, elle a pour objet l'utilisation d'au moins un composé de formule (I) dans des, ou pour la fabrication de, compositions cosmétiques pour la protection de la peau et/ou des cheveux contre le rayonnement ultraviolet, en particulier le rayonnement solaire.

La présente invention a également pour objet l'utilisation d'au moins un composé de formule (I) dans des, ou pour la fabrication de, vernis destinés à protéger des verres organiques et/ou minéraux ou des matières plastiques du rayonnement ultraviolet, en particulier du rayonnement solaire.

Les composés de l'invention peuvent également être incorporés directement dans des matières plastiques, ou dans d'autres matières sensibles au rayonnement ultraviolet, en vue de protéger ces dernières contre ledit rayonnement.

La présente invention a ainsi également pour objet un procédé de protection d'une matière sensible au rayonnement ultraviolet et/ou solaire contre ledit rayonnement, consistant à appliquer sur, ou à incorporer dans, ladite matière sensible une quantité efficace d'un composé de formule (I) ou d'une composition contenant au moins un composé de formule (I).

En particulier, le procédé selon l'invention peut consister à appliquer sur la peau et/ou les cheveux une quantité efficace d'une composition cosmétique telle que définie ci-dessus.

Dans une autre forme de réalisation de l'invention, le procédé selon l'invention consiste à incorporer dans une matière plastique une quantité efficace d'un composé de formule (I) ou d'une composition contenant au moins un composé de formule (I) afin de protéger ladite matière plastique contre le rayonnement ultraviolet, en particulier contre le rayonnement solaire.

Ainsi, un objet de l'invention est une composition de matière plastique protégée par un tel procédé.

Dans une autre forme de réalisation de l'invention, le procédé selon l'invention consiste à appliquer une quantité efficace dudit composé ou de ladite composition à la surface d'un verre minéral ou organique.

Ainsi, finalement, un dernier objet de l'invention est une composition verrière protégée par ce procédé.

Des exemples concrets, mais nullement limitatifs, illustrant l'invention, vont maintenant être donnés.

### EXEMPLE 1 :

### Préparation de la 2,4,6-tris(4'-amino benzalmalonate de diéthyle)-s-triazine :

On chauffe au reflux pendant 10 heures sous azote du chlorure de cyanuryle (0.92 g, 5x10⁻³ mole) et du 4-amino benzalmalonate de diéthyle (3.95 g, 15x10⁻³ mole) dans du xylène (50 ml). On refroidit, filtre et sèche le précipité obtenu (4.3 g, rendement = 60%). Après recristallisation dans l'éthanol absolu, on obtient le produit ayant les caractéristiques suivantes :
Poudre jaune clair insoluble
Pf : 130-140°C.
UV (Ethanol à 95) λₘₐₓ = 355 nm, εₘₐₓ = 99 400

| Analyse élémentaire pour C₄₅ H₄₈ N₆ O₁₆ | | | | | | | | |
|---|---|---|---|---|---|---|---|---|
| théorie | C | 62.49 | H | 5.59 | N | 9.72 | O | 22.20 |
| trouvé | C | 62.48 | H | 5.65 | N | 9.60 | O | 22.15 |

### EXEMPLE 2 :

### Préparation de la 2,4,6-tris(4'-amino benzalmalonate de diisopropyle)-s-triazine

On chauffe au reflux pendant 6 heures sous azote du chlorure de cyanuryle (2.12 g, 0.0115 mole) et du 4-amino benzalmalonate de diisopropyle (11 g, 0.0377 mole) dans du xylène (60 ml). On concentre sous vide et recristallise dans l'éthanol pour obtenir le produit (7.3 g, rendement = 67%) ayant les caractéristiques suivantes :
- poudre jaune clair insoluble
- Pf : 181 °C.
- UV (Ethanol à 95) λₘₐₓ = 355 nm, εₘₐₓ = 111 000

| Analyse élémentaire pour C₅₁ H₆₀ N₆ O₁₂ | | | | | | | | |
|---|---|---|---|---|---|---|---|---|
| théorie | C | 64.54 | H | 6.37 | N | 8.85 | O | 20.23 |
| trouvé | C | 64.76 | H | 6.41 | N | 8.80 | O | 20.09 |

Sur la figure 1 sont représentés sur une même échelle (Longueur d'onde en abscisse, Absorbance en ordonnée) le spectre UV de solide de la 2,4,6-tris(4'-amino benzalmalonate de diisopropyle)-s-triazine (courbe (1)) et le spectre UV dans l'éthanol du composé soluble chimiquement le plus proche, c'est-à-dire la 2,4,6-tris(4'-amino benzalmalonate de diisobutyle)-s-triazine (courbe (2)) dont la synthèse est décrite dans la demande de brevet au nom de la Demanderesse EP-A-0 507 691.

Les spectres ont été obtenus à partir d'un spectrophotomètre Shimadzu UV 2101 PC.

La 2,4,6-tris(4'-amino benzalmalonate de diisobutyle)-s-triazine a été solubilisée à une concentration de 5 mg/l dans de l'éthanol à 95%.

La 2,4,6-tris(4'-amino benzalmalonate de diisopropyle)-s-triazine brute a été tamisée à 50 µm sur un tamis de monture Inox-toile Inox. Puis elle a été dispersée dans de la vaseline blanche vendue sous la dénomination commerciale « Codex 236 » par la société Sarega à la température de fusion de la vaseline et à raison de 5 g de 2,4,6-tris(4'-amino benzalmalonate de diisopropyle)-s-triazine tamisée pour 100 g de vaseline. Ce mélange a ensuite été traité aux ultra-sons pour assurer une dispersion homogène. Un film d'une épaisseur de 60 µm a été analysé.

La figure 1 montre clairement que le composé insoluble selon la présente invention, à savoir la 2,4,6-tris(4'-amino benzalmalonate de diisopropyle)-s-triazine, absorbe dans l'ensemble du rayonnement UV (280-400 nm).

### EXEMPLE 3:

### Préparation de la 2,4,6-tris(α-cyano-4-aminocinnamate d'éthyle)-s-triazine :

On chauffe au reflux pendant 12 heures sous azote le chlorure de cyanuryle (1,1 g, 6 mmole) et le α-cyano-4-aminocinnamate d'éthyle (4,32 g, 0,02 mole) dans du xylène (100 ml). On filtre, lave la poudre jaune au xylène puis à l'éthanol pour obtenir le produit (3 g, rendement = 69 %) présentant les caractéristiques suivantes :
- poudre jaune d'or insoluble
- Pf : > 300°C
- UV (dmso) λₘₐₓ= 390 nm.
- Spectre UV solide (5 g de 2,4,6-tris(α-cyano-4-aminocinnamate d'éthyle)-s-triazine pour 100 g de vaseline « Codex 236 ») : cf. Fig 2.

### EXEMPLE 4 :

Un exemple de composition cosmétique se présentant sous la forme d'une émulsion de type huile-dans-eau va maintenant être donné (les quantités sont exprimées en % de poids par rapport au poids total de la composition) :
- mélange d'alcool cétylstéarylique et d'alcool cétylstéarylique oxyéthyléné à 33 moles d'oxyde d'éthylène vendu sous la dénomination commerciale « SINNOVAX AO » par Henkel 7 %
- mélange de mono et distéarate de glycérol non autoémulsionnable 2 %
- alcool cétylique 1,5 %
- huile de silicone 1,5 %
- adipate de diisopropyle 15 %
- 2,4,6-tris(4'-amino benzalmalonate de diéthyle)-s-triazine (filtre) 5 %
- glycérine 20 %
- parfum, conservateurs qs
- eau qsp 100 %

Cette composition a été réalisée de la manière suivante : après réalisation de l'émulsion, on a dispersé le filtre vers 40°C. On a ensuite homogénéisé la crème obtenue à la tricylindre.

## Revendications

1. Composé de formule (I) suivante : 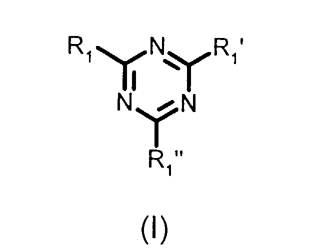 dans laquelle :
- R₁, R₁' et R₁", qui peuvent être identiques ou différents, sont choisis parmi les radicaux monovalents de formules A ou B suivantes : 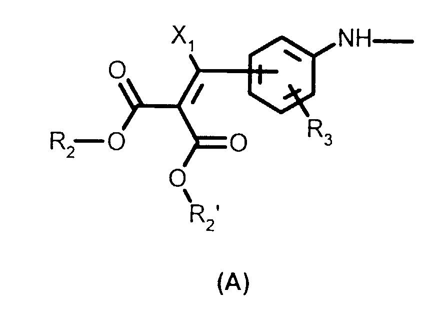 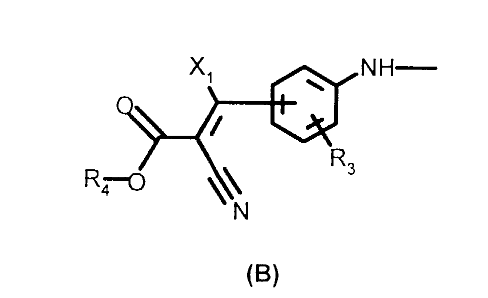 dans lesquelles :
- R₂ et R₂', qui peuvent être identiques ou différents, désignent un radical alkyle en C₁-C₃ linéaire ou ramifié,
- R₃ désigne un atome d'hydrogène, un radical alkyle en C₁-C₄ linéaire ou ramifié ou un radical alkoxy en C₁-C₄,
- R₄ désigne un radical alkyle en C₁-C₁₂ linéaire ou ramifié,
- X₁ représente un atome d'hydrogène ou un radical phényle éventuellement substitué par un halogène ou par un radical alkyle en C₁-C₄ ou par un radical alcoxy en C₁-C₄.

2. Composé selon la revendication 1, caractérisé par le fait que lesdits radicaux R₁, R₁' et R₁" sont identiques.

3. Composé selon la revendication 2, caractérisé par le fait que lesdits radicaux R₁, R₁' et R₁" identiques désignent chacun un radical de formule A.

4. Composé selon la revendication 3, caractérisé par le fait qu'il est choisi parmi les composés suivants :
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine.

5. Composé selon la revendication 2, caractérisé par le fait que lesdits radicaux R₁, R₁' et R₁" identiques désignent chacun un radical de formule B.

6. Composé selon la revendication 5, caractérisé par le fait qu'il s'agit de la 2,4,6-tris(α-cyano-4-aminocinnamate d'éthyle)-s-triazine.

7. Procédé de préparation d'un composé de formule (I) tel que défini à la revendication 1, caractérisé par le fait qu'il comprend le schéma réactionnel suivant : 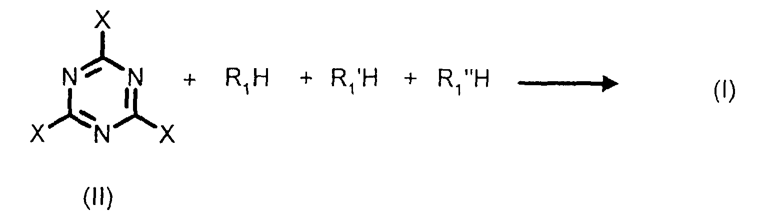 où R₁, R₁' et R₁" répondent aux définitions de la revendication 1 et X représente un halogène, en particulier le chlore ou le brome.

8. Procédé selon la revendication 7, caractérisé par le fait que, R₁, R₁' et R₁" étant différents, il est mis en oeuvre selon les trois étapes suivantes : i) R₁H est d'abord introduit dans le mélange réactionnel, ii) R₁'H est introduit lorsque R₁H a complètement réagi avec le composé de formule (II), iii) R₁"H est introduit lorsque R₁'H a complètement réagi avec le composé de formule (II) monosubstitué par R1.

9. Composition destinée à protéger une matière sensible au rayonnement ultraviolet, en particulier au rayonnement solaire, caractérisée par le fait qu'elle comprend une quantité efficace d'au moins un composé défini à l'une quelconque des revendications 1 à 6.

10. Composition selon la revendication 9, caractérisée par le fait qu'il s'agit d'une composition cosmétique destinée à protéger la peau et/ou les cheveux.

11. Composition selon la revendication 10, caractérisée par le fait que ledit composé est sous forme de particules.

12. Composition selon la revendication 11, caractérisée par le fait que la taille moyenne desdites particules est inférieure à 20 µm.

13. Composition selon l'une quelconque des revendications 10 à 12, caractérisée par le fait qu'elle contient de 0,1 à 15 % en poids, par rapport au poids total de la composition, dudit composé.

14. Composition selon l'une quelconque des revendications 10 à 13, caractérisée par le fait qu'elle contient en outre un ou plusieurs filtres solaires complémentaires actifs dans l'UVA et/ou l'UVB choisis parmi les dérivés cinnamiques, les dérivés salicyliques, les dérivés du camphre, les dérivés de triazine autres que ceux définis dans la revendication 1, les dérivés de la benzophénone, les dérivés du dibenzoylméthane, les dérivés de β,β-diphénylacrylate, les dérivés de l'acide p-aminobenzoïque, les polymères filtres et silicones filtres.

15. Composition selon l'une quelconque des revendications 10 à 14, caractérisée par le fait qu'elle se présente sous la forme d'une suspension ou d'une dispersion dans des solvants ou des corps gras, d'une dispersion vésiculaire non ionique ou encore d'une émulsion, de préférence de type huile-dans-eau, telle qu'une crème ou un lait, d'une pommade, d'un gel, d'un gel crème, d'un bâtonnet solide, d'un stick, d'une mousse aérosol ou d'un spray.

16. Composition selon la revendication 9, caractérisée par le fait qu'il s'agit d'un vernis destiné à protéger un verre organique et/ou minéral ou une matière plastique.

17. Utilisation d'au moins un composé défini à l'une quelconque des revendications 1 à 6 dans des, ou pour la fabrication de, compositions destinées à protéger des matières sensibles au rayonnement ultraviolet, en particulier au rayonnement solaire.

18. Utilisation selon la revendication 17, caractérisée par le fait que lesdites compositions sont des compositions cosmétiques pour la protection de la peau et/ou des cheveux contre le rayonnement ultraviolet, en particulier contre le rayonnement solaire.

19. Utilisation selon la revendication 17, caractérisée par le fait que lesdites compositions sont des vernis pour la protection des verres organiques et/ou minéraux ou des matières plastiques contre le rayonnement ultraviolet, en particulier contre le rayonnement solaire.

20. Procédé de protection d'une matière sensible au rayonnement ultraviolet et/ou solaire, caractérisé par le fait qu'il consiste à appliquer sur, ou à incorporer dans, ladite matière sensible une quantité efficace d'un composé tel que défini à l'une quelconque des revendications 1 à 6 ou d'une composition définie à l'une quelconque des revendications 9 à 16.

21. Procédé selon la revendication 20, caractérisé par le fait qu'il consiste à appliquer sur la peau et/ou les cheveux une quantité efficace d'une composition définie à l'une quelconque des revendications 10 à 15.

22. Procédé selon la revendication 20, caractérisé par le fait qu'il consiste à incorporer dans une matière plastique une quantité efficace dudit composé ou de ladite composition.

23. Procédé selon la revendication 20, caractérisé par le fait qu'il consiste à appliquer une quantité efficace dudit composé ou de ladite composition à la surface d'un verre minéral ou organique.

24. Une composition de matière protégée selon le procédé de l'une quelconque des revendications 20, 22 ou 23.

## Patentansprüche

1. Verbindung der folgenden Formel I 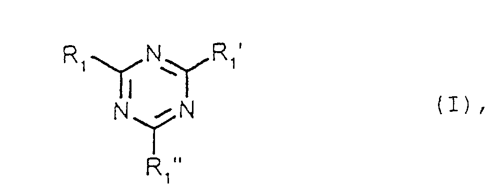 worin:
- die Gruppen R₁, R₁' und R₁", die identisch oder voneinander verschieder sein können, unter den einwernigen Gruppen der folgenden Formeln A oder B ausgewählt sind: 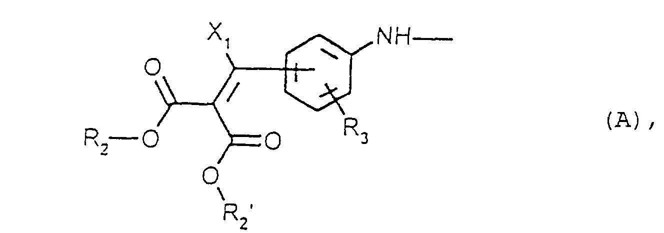 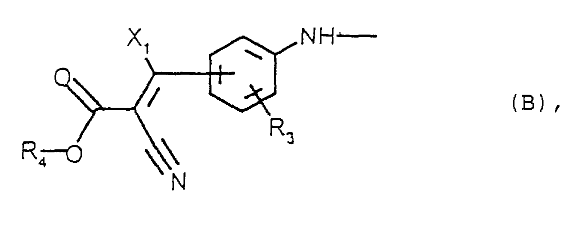
worin bedeuten:
- R₂ und R₂', die identisch oder voneinander verschieden sein können, eine geradkettige oder verzweigte C₁₋₃-Alkylgruppe,
- R₃ ein Wasserstoffatom, eine geradkettige oder verzweigte C₁₋₄-Alkylgruppe oder eine C₁₋₄-Alkoxygruppe,
- R₄ eine geradkettige oder verzweigte C₁₋₁₂-Alkylgruppe,
- X₁ ein Wasserstoffatom oder eine Phenylgruppe, die gegebenenfalls mit einem Halogen oder einer C₁₋₄-Alkylgruppe oder einer C₁₋₄-Alkoxygruppe substituiert ist.

2. Verbindung nach Anspruch 1, dadurch gekennzeichnet, daß die Gruppen, R₁, R₁' und R₁" identisch sind.

3. Verbindung nach Anspruch 2, dadurch gekennzeichnet, daß die Gruppen R₁, R₁' und R₁" identisch sind und jeweils eine Gruppe der Formel A bedeuten.

4. Verbindung nach Anspruch 3, dadurch gekennzeichnet, daß sie unter den folgenden Verbindungen ausgewählt ist
- 2,4,6-Tris(diethyl-4'-aminobenzalmalonat)-s-triazin,
- 2,4,6-Tris(diisopropyl-4'-aminobenzalmalonat)-s-triazin,
- 2,4,6-Tris(dimethyl-4'-aminobenzalmalonat)-s-triazin.

5. Verbindung nach Anspruch 2, dadurch gekennzeichnet, daß die Gruppen R₁, R₁' und R₁" identisch sind und jeweils eine Gruppe der Formel B bedeuten.

6. Verbindung nach Anspruch 5, dadurch gekennzeichnet, daß es sich um 2,4,6-Tris(ethyl-α-cyano-4-aminocinnamat)-s-triazin handelt.

7. Verfahren zur Herstellung einer Verbindung der Formel I nach Anspruch 1, dadurch gekennzeichnet, daß es das folgende Reaktionsschema umfaßt: 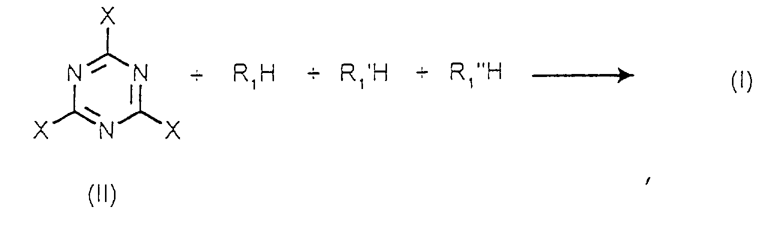 worin R₁, R₁' und R₁" die in Anspruch 1 angegebenen Bedeutungen aufweisen und X ein Halogenatom bedeutet, insbesondere Chlor oder Brom.

8. Verfahren nach Anspruch 7, dadurch gekennzeichnet, daß die Gruppen R₁, R₁' und R₁", die voneinader verschieden sind, gemäß den drei folgenden Schritten eingesetzt werden:
i) zunächst wird R₁H in das Reaktionsgemisch gegeben,
ii) R₁'H wird zugegeben, wenn R₁H vollständig mit der Verbindung der Formel II reagiert hat und
iii) R₁"H wird zugegeben, wenn R₁'H vollständig mit der Verbindung der Formel II, die mit R₁ einfach substituiert ist, reagiert hat.

9. Zusammensetzung zum Schutz eines gegenüber UV-Strahlung und insbesondere gegenüber Sonnenlicht empfindlichen Materials, dadurch gekennzeichnet, daß sie eine wirksame Menge mindestens einer Verbindung nach einem der Ansprüche 1 bis 6 enthält.

10. Zusammensetzung nach Anspruch 9, dadurch gekennzeichnet, daß es sich um eine kosmetische Zusammensetzung zum Schutz der Haut und/oder der Haare handelt.

11. Zusammensetzung nach Anspruch 10, dadurch gekennzeichnet, daß die Verbindung in Partikelform vorliegt.

12. Zusammensetzung nach Anspruch 11, dadurch gekennzeichnet, daß die mittlere Größe der Partikel unter 20 µm liegt.

13. Zusammensetzung nach einem der Ansprüche 10 bis 12, dadurch gekennzeichnet, daß sie 0,1 bis 15 Gew.-% der Verbindung, bezogen auf das Gesamtgewicht der Zusammensetzung, enthält.

14. Zusammensetzung nach einem der Ansprüche 10 bis 13, dadurch gekennzeichnet, daß sie ferner ein oder mehrere, zusätzliche, im UV-A- und/oder UV-B-Bereich wirksame Filter enthält, die unter den Zimtsäurederivaten, Salicylsäurederivaten, Campherderivaten, Triazinderivaten, die von den in Anspruch 1 definierten Derivaten verschieden sind, Benzophenonderivaten, Dibenzoylmethanderivaten, β,β-Diphenylacrylatderivaten, p-Aminobenzoesäurederivaten, polymeren Filtern und Siliconfiltern ausgewählt sind.

15. Zusammensetzung nach einem der Ansprüche 10 bis 14, dadurch gekennzeichnet, daß sie in Form einer Suspension oder Dispersion in Lösungsmitteln oder Fettsubstanzen, in Form einer nichtionischen Vestikeldispersion oder in Form einer Emulsion, vorzugsweise vom Öl-in-Wasser-Typ, wie beispielsweise als Creme oder Milch, in Form einer Pomade, eines Gels, einer Gelcreme, eines festen Stifts, eines Sticks, eines Aerosolschaums oder eines Sprays vorliegt.

16. Zusammensetzung nach Anspruch 9, dadurch gekennzeichnet, daß es sich um einen Lack zum Schutz eines organischen und/oder anorganischen Glases oder Kunstsoffmaterials handelt.

17. Verwendung mindestens einer Verbindung nach einem der Ansprüche 1 bis 6 in Zusammensetzungen zum Schutz von gegenüber UV-Strahlung und insbesondere gegenüber Sonnenlicht empfindlichen Materialien oder zur Herstellung dieser Zusammensetzungen.

18. Verwendung nach Anspruch 17, dadurch gekennzeichnet, daß die Zusammensetzungen kosmetische Zusammensetzungen zum Schutz der Haut und/oder der Haare gegen UV-Strahlung und insbesondere gegen Sonnenlicht sind.

19. Verwendung nach Anspruch 17, dadurch gekennzeichnet, daß die Zusammensetzungen Lacke zum Schutz von organischen und/oder anorganischen Gläsern oder Plastikmaterialien gegen UV-Strahlung und insbesondere gegen Sonnenlicht sind.

20. Verfahren zum Schutz eines gegenüber UV-Strahlung und/oder Sonnenlicht empfindlichen Materials, dadurch gekennzeichnet, daß es darin besteht, eine wirksame Menge einer Verbindung nach einem der Ansprüche 1 bis 6 oder einer Zusammensetzung nach einem der Ansprüche 9 bis 16 auf das empfindliche Material aufzutragen oder in das empfindliche Material einzuarbeiten.

21. Verfahren nach Anspruch 20, dadurch gekennzeichnet, der es darin besteht, auf die Haut und/oder die Haare eine wirksame Menge einer Zusammensetzung nach einem der Ansprüche 10 bis 15 aufzutragen.

22. Verfahren nach Anspruch 20, dadurch gekennzeichnet, daß es darin besteht, eine wirksame Menge der Verbindung oder der Zusammensetzung in ein Kunststoffmaterial einzuarbeiten.

23. Verfahren nach Anspruch 20, dadurch gekennzeichnet, daß es darin besteht, eine wirksame Menge der Verbindung oder der Zusammensetzung auf die Oberfläche eines anorganischen oder organischen Glases aufzutragen.

24. Zusammensetzung aus einem Material, das nach dem Verfahren nach einem der Ansprüche 20, 22 oder 23 geschützt ist.

## Claims

1. Compound of formula (I) below: 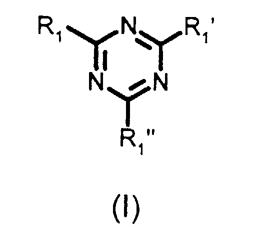 in which:
- R₁, R₁' and R₁", which may be identical or different, are chosen from monovalent radicals of formulae A or B below: 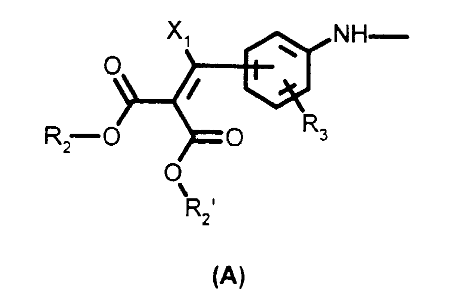 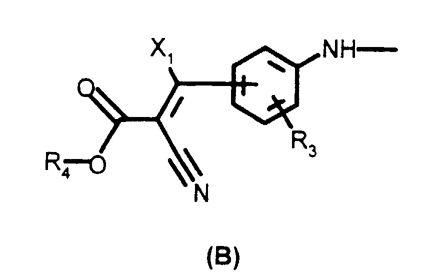
in which:
- R₂ and R₂', which may be identical or different, denote a linear or branched C₁-C₃ alkyl radical,
- R₃ denotes a hydrogen atom, a linear or branched C₁-C₄ alkyl radical or a C₁-C₄ alkoxy radical,
- R₄ denotes a linear or branched C₁-C₁₂ alkyl radical,
- X₁ represents a hydrogen atom or a phenyl radical optionally substituted with a halogen or with a C₁-C₄ alkyl radical or with a C₁-C₄ alkoxy radical.

2. Compound according to Claim 1, characterized in that the said radicals R₁, R₁' and R₁" are identical.

3. Compound according to Claim 2, characterized in that the said identical radicals R₁, R₁' and R₁" each denote a radical of formula A.

4. Compound according to Claim 3, characterized in that it is chosen from the following compounds:
- 2,4,6-tris(diethyl 4'-aminobenzalmalonate)-s-triazine,
- 2,4,6-tris(diisopropyl 4'-aminobenzalmalonate)-s-triazine,
- 2,4,6-tris(dimethyl 4'-aminobenzalmalonate)-s-triazine.

5. Compound according to Claim 2, characterized in that the said identical radicals R₁, R₁' and R₁" each denote a radical of formula B.

6. Compound according to Claim 5, characterized in that it is 2,4,6-tris(ethyl α-cyano-4-aminocinnamate)-s-triazine.

7. Process for the preparation of a compound of formula (I) as defined in Claim 1, characterized in that it comprises the following reaction scheme: 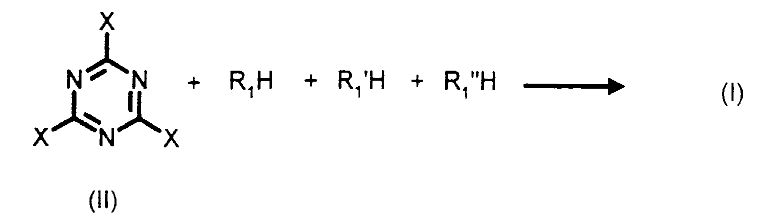 where R₁, R₁' and R₁" correspond to the definitions of Claim 1 and X represents a halogen, in particular chlorine or bromine.

8. Process according to Claim 7, characterized in that, with R₁, R₁' and R₁" being different, it is carried out according to the following three steps: i) R₁H is first introduced into the reaction mixture, ii) R₁'H is introduced when R₁H has completely reacted with the compound of formula (II), iii) R₁"H is introduced when R₁'H has completely reacted with the compound of formula (II) monosubstituted with R₁.

9. Composition intended to protect a material which is sensitive to ultraviolet radiation, in particular to solar radiation, characterized in that it comprises an effective amount of at least one compound defined in any one of Claims 1 to 6.

10. Composition according to Claim 9, characterized in that it is a cosmetic composition intended to protect the skin and/or the hair.

11. Composition according to Claim 10, characterized in that the said compound is in the form of particles.

12. Composition according to Claim 11, characterized in that the average size of the said particles is less than 20 µm.

13. Composition according to any one of Claims 10 to 12, characterized in that it contains from 0.1 to 15 % by weight of the said compound relative to the total weight of the composition.

14. Composition according to any one of Claims 10 to 13, characterized in that it also contains one or more complementary sunscreens which are active in the UVA range and/or the UVB range, chosen from cinnamic derivatives, salicylic derivatives, camphor derivatives, triazine derivatives other than those defined in Claim 1, benzophenone derivatives, dibenzoylmethane derivatives, β,β-diphenylacrylate derivatives, p-aminobenzoic acid derivatives, screening polymers and screening silicones.

15. Composition according to any one of Claims 10 to 14, characterized in that it is in the form of a suspension or a dispersion in solvents or fatty substances, a nonionic vesicle dispersion or alternatively an emulsion, preferably of oil-in-water type, such as a cream or a milk, or in the form of an ointment, a gel, a cream gel, a solid stick, a pencil, an aerosol foam or a spray.

16. Composition according to Claim 9, characterized in that it is a varnish intended to protect an organic and/or inorganic glass or a plastic.

17. Use of at least one compound defined in any one of Claims 1 to 6 in, or for the manufacture of, compositions intended to protect materials which are sensitive to ultraviolet radiation, in particular solar radiation.

18. Use according to Claim 17, characterized in that the said compositions are cosmetic compositions for protecting the skin and/or the hair against ultraviolet radiation, in particular against solar radiation.

19. Use according to Claim 17, characterized in that the said compositions are varnishes for protecting organic and/or inorganic glasses or plastics against ultraviolet radiation, in particular against solar radiation.

20. Process for protecting a material which is sensitive to ultraviolet and/or solar radiation, characterized in that it consists in applying to, or incorporating into, the said sensitive material an effective amount of a compound as defined in any one of Claims 1 to 6 or of a composition defined in any one of Claims 9 to 16.

21. Process according to Claim 20, characterized in that it consists in applying an effective amount of a composition defined in any one of Claims 10 to 15 to the skin and/or the hair.

22. Process according to Claim 20, characterized in that it consists in incorporating an effective amount of the said compound or of the said composition into a plastic.

23. Process according to Claim 20, characterized in that it consists in applying an effective amount of the said compound or of the said composition to the surface of an inorganic or organic glass.

24. A composition of material protected according to the process of any one of Claims 20, 22 and 23.
